# FASCICULE DE BREVET EUROPEEN

(11) **EP 3 520 089 B1**
(45) Date de publication et mention de la délivrance du brevet: **11.03.2020**
(21) Numéro de dépôt: 16785468.6
(22) Date de dépôt: 24.10.2016
(51) Int. Cl.: G07F 11/60, G07F 11/62, G07G 1/00, G07F 17/00

(54) **DISPOSITIF DE STOCKAGE D'ÉLÉMENTS**
VORRICHTUNG ZUR AUFBEWAHRUNG VON ELEMENTEN
DEVICE FOR STORING ELEMENTS

(30) Priorité: 03.10.2016 FR 1659518
(43) Date de publication de la demande: 07.08.2019
(73) Titulaire: BIOLOG-ID, 75008 Paris (FR)
(72) Inventeur: MONGRENIER, Jean-Claude, 78000 Versailles (FR)
(74) Mandataire: Lavoix
(86) Numéro de dépôt international: PCT/EP2016/075498
(87) Numéro de publication internationale: WO 2018/065075

(56) Documents cités:
- WO-A1-03/026724
- CA-A1- 2 545 978
- FR-A1- 2 988 936
- US-A- 5 839 806
- US-A1- 2011 140 831
- US-B1- 8 770 479

## Description

La présente invention concerne un dispositif de stockage d'éléments.

La présente invention se rapporte également à une installation comprenant un tel dispositif de stockage.

Les éléments sont, par exemple, des poches contenant des produits biologiques, tels que des produits sanguins (poches de sang primaire, de plasma, de plaquettes, de globules rouges...) ou des produits d'ingénieries cellulaires (cellules, souches...), ou encore des poches de médicaments, telles que des poches de chimiothérapie.

Il est connu de stocker de telles poches dans des structures réfrigérantes formées de tiroirs dans lesquels les poches sont insérées. Les poches insérées dans de telles structures comprennent généralement une étiquette identificatrice, telle qu'une étiquette RFID (de l'anglais « radio frequency identification » traduit en français par « radio identification »), dans laquelle sont mémorisées des informations relatives à la poche correspondante. En outre, un lecteur, tel qu'un lecteur RFID, est disposé en vis-à-vis de l'emplacement prévu des poches de chaque tiroir afin de lire et de mettre à jour les informations contenues dans les étiquettes desdites poches.

Toutefois, lorsque les étiquettes des poches ne sont pas positionnées directement en vis-à-vis du lecteur dans le tiroir, la lecture des informations contenues dans l'étiquette desdites poches est susceptible de ne pas être effectuée.

De tels dispositifs de stockage d'éléments sont par exemple connus des documents FR 2988936 A1 et US 8770479 A.

Il existe donc un besoin pour un dispositif de stockage d'éléments permettant de contrôler l'état desdits éléments de manière fiable sans encombrer l'espace de stockage.

A cet effet, l'invention a pour objet un dispositif de stockage d'éléments selon la revendication 1.

Suivant des modes de réalisation particuliers, le dispositif comprend une ou plusieurs des caractéristiques des revendications 2 à 8, prises isolément ou suivant toutes les combinaisons techniquement possibles.

L'invention concerne aussi une installation selon la revendication 9.

Suivant des modes de réalisation particuliers, l'installation comprend une ou plusieurs des caractéristiques des revendications 10 et 11, prises isolément ou suivant toutes les combinaisons techniquement possibles.

D'autres caractéristiques et avantages de l'invention apparaîtront à la lecture de la description qui suit de modes de réalisation de l'invention, donnée à titre d'exemple uniquement et en référence aux dessins qui sont :
- figure 1, une représentation schématique en perspective d'une installation comprenant un dispositif de stockage,
- figure 2, une représentation schématique en perspective du dispositif de stockage de la figure 1,
- figure 3, une représentation schématique de plusieurs blocs tiroirs du dispositif de stockage de la figure 1, et
- figure 4, une représentation schématique d'un élément destiné à être stocké dans le dispositif de la figure 1.

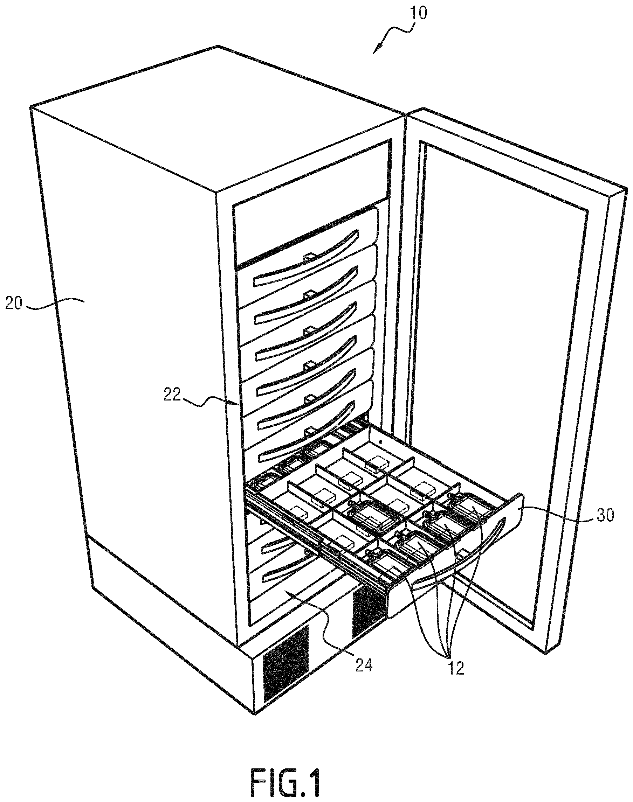

Une installation 10 de stockage d'éléments 12 est illustrée sur la figure 1.

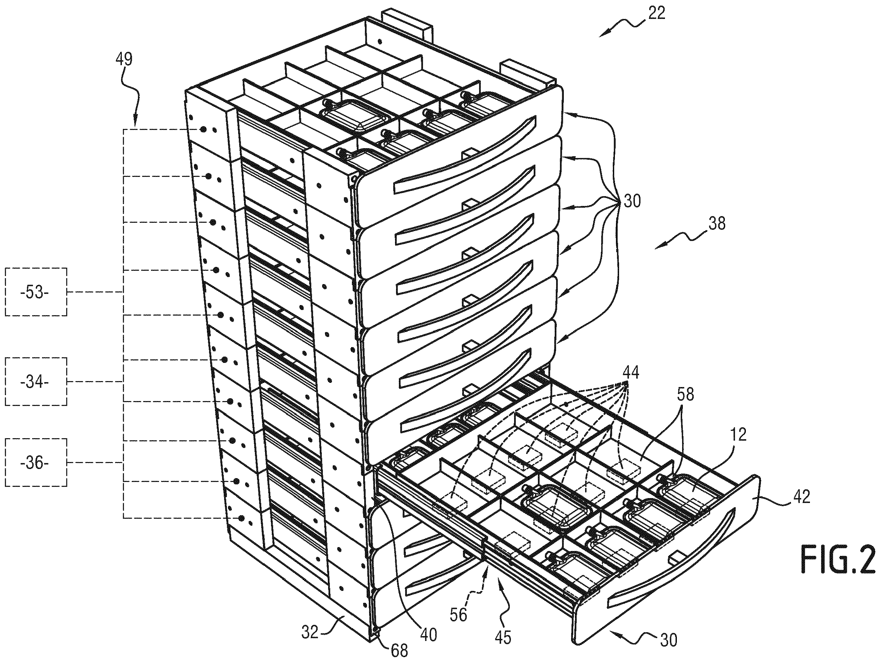

Les éléments 12 sont, par exemple, des conteneurs (visibles sur la figure 2). De manière générale, un conteneur désigne tout type de poche destiné à contenir des produits dont l'utilisation est conditionnée par des contraintes de stockage strictes.

Plus particulièrement, les éléments 12 sont, par exemple, des poches contenant des produits biologiques tels que des produits sanguins (poches de sang primaire, de plasma, de plaquettes, de globules rouges...) ou des produits d'ingénierie cellulaires (cellules humaines ou animales, notamment cellules souches humaines ou animales, produits issus de cellules humaines ou animales).

En variante, les éléments 12 sont des poches de médicaments ou des préparations thérapeutiques contenant un ou plusieurs principes actifs ou médicaments, telles que des poches de chimiothérapie contenant généralement un soluté et un ou plusieurs principes actifs de chimiothérapie.

De manière plus générale, les éléments 12 sont propres à contenir tout produit destiné à être perfusé à un humain ou à un animal.

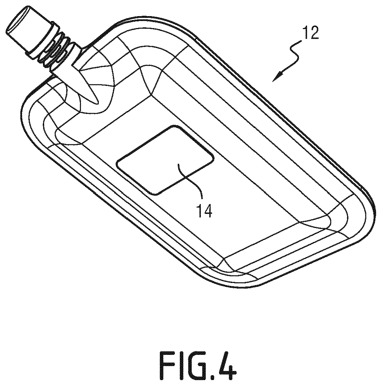

Comme visible sur la figure 4, chaque élément 12 comprend une première unité de communication sans fil 14. Chaque première unité de communication 14 est, par exemple, une étiquette, telle qu'une étiquette adhésive fixée sur une face extérieure de l'élément 12.

De manière générale, chaque première unité de communication 14 comprend au moins une antenne, une mémoire et, éventuellement, un micro-processeur.

L'antenne de chaque première unité de communication 14 est, par exemple, une antenne radiofréquence.

La mémoire de chaque première unité de communication 14 comprend des informations relatives à l'élément 12 correspondant.

De telles informations sont, par exemple : un identifiant unique de l'élément 12, la date de stockage de l'élément 12, la date de péremption de l'élément 12, la date à laquelle la première unité de communication 14 de l'élément 12 a communiqué des informations pour la dernière fois, le numéro du don relatif au contenu de l'élément 12, le code produit du contenu de l'élément 12, le groupe rhésus du contenu de l'élément 12, le phénotype sanguin du contenu de l'élément 12, l'identité du patient dont provient le contenu de l'élément 12, le nom du patient dont provient le contenu de l'élément 12, le volume du contenu de l'élément 12, le centre de don (y compris l'adresse) où a été obtenu le contenu de l'élément 12, le processus en cours sur l'élément 12 et le type d'anticoagulant du contenu de l'élément 12. Dans le cas d'une chimiothérapie, de telles informations comprennent, en outre, la date de fabrication, le type de produit, le type de véhicule, l'identité du médecin prescripteur, l'identité du pharmacien, l'identité du fabricant, la date de libération et le statut (libéré, délivré, etc).

L'installation 10 comprend une enceinte 20 et un dispositif de stockage 22.

L'enceinte 20 comprend un compartiment interne 24 de réception du dispositif 22 de stockage.

L'enceinte 20 est, par exemple, une enceinte réfrigérante, telle qu'un réfrigérateur ou un congélateur. Lorsque l'enceinte réfrigérante est un réfrigérateur, la température de l'enceinte est comprise entre 0 degrés Celsius (°C) et 5°, de préférence égale à 4°C. Lorsque l'enceinte réfrigérante est un congélateur, la température de l'enceinte est comprise entre -35°C et -196°C, de préférence égale à - 40°C.

En variante, l'enceinte 20 est un agitateur de plaquettes. L'enceinte 20 est alors de préférence intégrée dans un incubateur ayant une température, de préférence égale à 24°C.

Dans ce qui suit, il est défini des positionnements relatifs par rapport à un sens courant d'utilisation de l'enceinte 20 pour laquelle il est défini un bas reposant généralement sur le sol et un haut à l'opposé du bas. Ces positionnements relatifs sont notamment mis en évidence par des termes comme « en-dessous » ou « au-dessus ».

Le dispositif 22 comprend une pluralité de blocs tiroirs 30 et une base 32. Comme visible sur la figure 2, le dispositif 22 comprend, en outre, une unité de traitement 33.

Comme cela est décrit plus tard, les blocs tiroirs 30 sont empilés les uns sur les autres pour former un empilement vertical 38 de blocs tiroirs 30. Les figures 1 à 3 illustrent un exemple d'un empilement de dix blocs tiroirs 30.

Chaque bloc tiroir 30 comprend un support 40, un tiroir 42 et au moins une deuxième unité de communication 44, visible sur la figure 2.

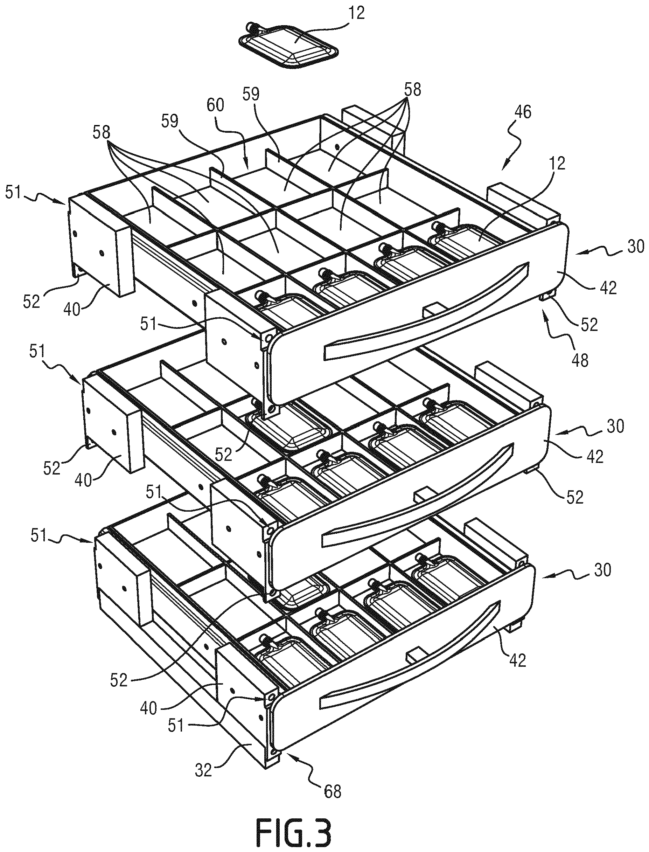

Le support 40 comprend un logement 45, une extrémité supérieure 46, une extrémité inférieure 48 (visibles sur la figure 3) et des connexions 49 (visibles sur la figure 2). Optionnellement, chaque bloc tiroir 30 comprend, également, une plaque.

Chaque logement 45 est propre à recevoir le tiroir 42 correspondant.

L'extrémité supérieure 46 de chaque bloc tiroir 30, visible sur la figure 3, comprend au moins un premier organe d'assemblage 51. Les premiers organes d'assemblage 51 sont, par exemple, des organes d'assemblage femelles.

L'extrémité inférieure 48 de chaque bloc tiroir 30, visible sur la figure 3, comprend au moins un deuxième organe d'assemblage 52, complémentaire des premiers organes d'assemblage 51. Les deuxièmes organes d'assemblage 52 sont, par exemple, des organes d'assemblage mâles.

Dans l'exemple illustré sur la figure 3, les premiers organes d'assemblage 51 sont des fentes et les deuxièmes organes d'assemblage 52 sont des nervures complémentaires des fentes.

Ainsi, chaque bloc tiroir 30 est assemblé à au moins un autre bloc tiroir 30 de l'empilement 38 par le ou les premier(s) organe(s) d'assemblage 51 dudit bloc tiroir 30 et/ou par le ou les deuxième(s) organe(s) d'assemblage 52 dudit bloc tiroir 30.

Les connexions 49 sont, par exemple, des connexions électriques.

Dans le mode de réalisation illustré sur les figures 1 à 3, les connexions 49 de chaque bloc tiroir 30 sont connectées, d'une part, aux deuxièmes unités de communication 44 dudit bloc tiroir 30, et d'autre part, aux deuxièmes unités de communication 44 des autres blocs tiroirs 30. En outre, les connexions 49 sont connectées à l'unité de traitement 33.

Chaque tiroir 42 est positionné dans le logement 45 du support 40. Chaque tiroir 42 est propre à coulisser par rapport au support 40 correspondant.

Chaque tiroir 42 comprend un fond 56 définissant au moins un emplacement 58 de réception d'un élément 12.

Le fond 56 de chaque tiroir 42 est formé d'un matériau apte à être traversé par des ondes radioélectriques émises par la deuxième unité de communication 44 du bloc tiroir 30 dudit tiroir 42.

Le matériau du fond 56 de chaque tiroir 42 est, par exemple, du plastique.

Dans le mode de réalisation illustré sur les figures 1 à 3, le fond 56 de chaque tiroir 42 définit douze emplacements 58 de réception d'éléments 12.

Chaque emplacement 58 est, par exemple, délimité par des rebords 59 formant un casier 60.

Dans le mode de réalisation illustré sur les figures 1 à 3, chaque bloc tiroir 30 comprend autant de deuxièmes unités de communication 44 que d'emplacements 58.

Chaque deuxième unité de communication 44 est disposée en-dessous du fond 56 du tiroir 42 en vis-à-vis de l'emplacement 58 correspondant, de sorte à permettre la communication entre ladite deuxième unité de communication 44 et la première unité de communication 14 d'un élément 12 reçu dans ledit emplacement 58. Il est entendu par l'expression « en vis-à-vis de », le fait que chaque deuxième unité de communication 44 est disposée en face de l'espace délimité par l'emplacement 58. Autrement formulé, la projection de l'emplacement 58 dans le plan de la deuxième unité de communication 44 est confondue avec la deuxième unité de communication 44.

Chaque deuxième unité de communication 44 est propre à communiquer, le cas échéant, avec la première unité de communication 14 de l'élément 12 reçu dans ledit emplacement 58 pour obtenir des informations relatives à l'élément 12.

Chaque deuxième unité de communication 44 est propre à émettre des ondes radiofréquences. Chaque deuxième unité de communication 44 est adaptée pour communiquer avec l'ensemble des premières unités de communication 14.

Dans un exemple de réalisation, les premières unités de communication 14 sont des étiquettes RFID et les deuxièmes unités de communication 44 sont des lecteurs RFID.

Plus généralement, chaque deuxième unité de communication 44 comprend au moins une antenne, une mémoire et, éventuellement, un micro-processeur.

Dans le mode de réalisation illustré sur les figures 1 à 3, chaque deuxième unité de communication 44 est solidaire du tiroir 42 du bloc tiroir 30 correspondant. Plus précisément, chaque deuxième unité de communication 44 est fixée en dessous du fond 56 du tiroir 42 de l'emplacement 58 correspondant.

Dans une variante, chaque deuxième unité de communication 44 est solidaire du support 40 du bloc tiroir 30 correspondant. Chaque bloc tiroir 30 comprend, en outre, un satellite. Le satellite est un boîtier qui contient la deuxième unité de communication 44. Le satellite est fixé au support 40 dudit bloc tiroir 30 directement sous le tiroir 42 dudit bloc tiroir 30. Quand le tiroir 42 est fermé, ladite deuxième unité de communication 44 est en face de l'emplacement 58 correspondant et est donc, le cas échéant, capable de communiquer avec une première unité de communication 14 positionnée dans l'emplacement 58 correspondant. Quand le tiroir 42 est ouvert, ladite deuxième unité de communication 44 n'est pas déplacée avec le tiroir 42, et par conséquent, n'est pas capable de communiquer avec une première unité de communication 14 positionnée dans l'emplacement 58 correspondant.

Chaque plaque est propre à empêcher le passage d'ondes radioélectriques émises par toutes deuxièmes unités de communication 44.

Chaque plaque est positionnée en-dessous du fond 56 du tiroir 42 de chaque bloc tiroir 30 et en-dessous des deuxièmes unités de communication 44 correspondant aux emplacements 58 du fond 56 du tiroir 42 dudit bloc tiroir 30. Ainsi, chaque deuxième unité de communication 44 est propre à communiquer uniquement avec les premières unités de communication 14 positionnées au-dessus de ladite deuxième unité de communication 44.

Chaque plaque est, par exemple, réalisée en métal.

La base 32 est assemblée avec le bloc tiroir 30 le plus bas de l'empilement 38 de blocs tiroirs 30. A cet effet, la base 32 comprend une extrémité supérieure 62 comprenant au moins un troisième organe d'assemblage. Chaque troisième organe d'assemblage est identique aux premiers organes d'assemblage 51. Le ou les deuxièmes organes d'assemblage 52 du dernier bloc tiroir 30 de l'empilement 38 sont assemblés avec le ou les troisièmes organes d'assemblage de la base 32, ce qui permet de clore l'empilement 38.

L'unité de traitement 33 est propre à traiter les informations en provenance des deuxièmes unités de communication 44. En particulier, l'unité de traitement 33 est propre à déterminer l'occupation de chaque emplacement 58 et, le cas échéant, à partir desdites informations, un état de l'élément 12 positionné dans ledit emplacement 58.

Les états déterminés sont, par exemple, au nombre de deux : un état « valide » et un état « invalide ». Un élément 12 est considéré comme « valide » lorsque les informations relatives à l'élément 12 sont conformes à un cahier des charges et est considéré « invalide » dans le cas contraire.

Ainsi, l'unité de traitement 33 a une image instantanée du dispositif 22 de stockage, à savoir quel élément 12 est dans quel emplacement 58 et les informations relatives à chacun desdits éléments 12. L'unité de traitement 33 comprend également un historique des dates d'entrée et de sortie de chaque élément 12 par rapport au dispositif 22.

En outre, l'unité de traitement 33 est propre à coordonner les deuxièmes unités de communication 44. En particulier, l'unité de traitement 33 est propre à activer chaque deuxième unité de communication 44 et à ordonner, le cas échéant, la mise à jour, par les deuxièmes unités de communication 44, des informations contenues dans les premières unités de communication 14.

Optionnellement, l'unité de traitement 33 est propre à générer une alarme en fonction de l'occupation de chaque emplacement 58 et, le cas échéant, de l'état de l'élément 12 correspondant audit emplacement 58. Par exemple, si l'unité de traitement 33 détermine qu'un même emplacement 58 comprend plus d'un élément 12, l'unité de traitement 33 génère une alarme.

Le fonctionnement du dispositif 22 intégré dans l'installation 10 va maintenant être décrit.

Lorsqu'un élément 12 est positionné dans l'emplacement 58 de l'un des blocs tiroirs 30 du dispositif 22, la deuxième unité de communication 44 correspondant audit emplacement 58 communique avec la première unité de communication 14 dudit élément 12, pour obtenir des informations relatives à l'élément 12. A partir des informations recueillies, l'unité de traitement 33 détermine un état de chaque élément 12, et, le cas échéant, déclenche ou non une alarme.

Ainsi, le dispositif 22 permet de contrôler de manière fiable l'état des éléments 12 stockés dans le dispositif 22, de même que le taux d'occupation des emplacements 58 des blocs tiroirs 30.

De plus, le positionnement spécifique des lecteurs en-dessous du tiroir correspondant permet d'obtenir un encombrement réduit.

Le dispositif 22 est donc un dispositif de stockage d'éléments permettant de contrôler l'état desdits éléments 12 de manière fiable sans encombrer l'espace de stockage.

En outre, il est aisé d'assembler et de désassembler les blocs tiroirs 30 du dispositif 22. Une telle modularité du dispositif 22 permet d'adapter le dispositif 22 à un grand nombre d'installations 10, en modifiant le nombre de blocs tiroirs 30 de l'empilement 38.

De plus, un empilement de blocs tiroirs est beaucoup moins lourd et encombrant qu'une pluralité de tiroirs, donc plus simple à manipuler et à installer.

En outre, d'un point de vue fabrication, des milliers de blocs tiroirs 30 identiques sont fabriquées, plutôt que des dizaines d'armoires de format différent. Cela permet d'effectuer des économies d'échelle, de simplifier la gestion du stock, ainsi que la maintenance.

Par ailleurs, le dispositif 22 est adaptable sur une installation 10 ne disposant pas préalablement de la technologie RFID.

En outre, l'encombrement réduit permet d'envisager des configurations dans lesquelles l'installation 10 contient un plus grand nombre d'éléments 12.

De plus, l'installation 10 et/ou le dispositif 22 sont aisés à fabriquer.

Enfin, dans la variante selon laquelle chaque bloc tiroir 30 comprend un satellite, le satellite est en seul bloc et est donc aisé à remplacer en cas de panne.

## Revendications

1. Dispositif (22) de stockage d'éléments (12), chaque élément (12) comprenant une première unité de communication sans fil (14), le dispositif (22) comprenant au moins un bloc tiroir (30), chaque bloc tiroir (30) comprenant :
- un support (40) comprenant un logement (45),
- un tiroir (42) positionné dans le logement (45) du support (40) et propre à coulisser par rapport au support (40), le tiroir (42) comprenant un fond (56) définissant au moins un emplacement (58) de réception d'un élément (12),
- pour chaque emplacement (58), au moins une deuxième unité de communication sans fil (44) propre à émettre des ondes radiofréquences, la deuxième unité de communication (44) étant adaptée pour communiquer avec l'ensemble des premières unités de communication (14),
**caractérisé en ce que** le fond (56) du tiroir (42) étant formé d'un matériau apte à être traversé par des ondes radiofréquences émises par la ou chaque deuxième unité de communication (44), la ou chaque deuxième unité de communication (44) étant disposée en-dessous du fond (56) du tiroir (42) en vis-à-vis de l'emplacement (58) correspondant du fond (56) du tiroir (42) de sorte à permettre la communication entre ladite deuxième unité de communication (44) et la première unité de communication (14) d'un élément (12) reçu dans ledit emplacement (58).

2. Dispositif (22) selon la revendication 1, dans lequel le fond (56) du tiroir (42) est en plastique.

3. Dispositif (22) selon la revendication 1 ou 2, dans lequel les éléments (12) sont des conteneurs de produits biologiques, de médicaments ou de préparations thérapeutiques.

4. Dispositif (22) selon l'une quelconque des revendications 1 à 3, dans lequel chaque première unité de communication (14) est une étiquette de radio-identification et chaque deuxième unité de communication (44) est un lecteur de radio-identification.

5. Dispositif (22) selon l'une quelconque des revendications 1 à 4, dans lequel chaque deuxième unité de communication (44) est solidaire du support (40) du bloc tiroir (30) correspondant.

6. Dispositif (22) selon l'une quelconque des revendications 1 à 4, dans lequel chaque deuxième unité de communication (44) est solidaire du tiroir (42) du bloc tiroir (30) correspondant.

7. Dispositif (22) selon l'une quelconque des revendications 1 à 6, dans lequel le dispositif (22) comprend une pluralité de blocs tiroirs (30), une extrémité supérieure (46) et/ou une extrémité inférieure (48) du support (40) de chaque bloc tiroir (30) comprenant un organe d'assemblage (51, 52) avec un organe d'assemblage (51, 52) complémentaire, respectivement, d'une extrémité inférieure (48) ou d'une extrémité supérieure (48) du support (40) d'un autre desdits blocs tiroirs (30) de sorte que les blocs tiroirs (30) sont assemblés les uns sur les autres pour former un empilement vertical (38).

8. Dispositif (22) selon l'une quelconque des revendications 1 à 7, dans lequel chaque première unité de communication (14) comprend des informations relatives à l'élément (12) correspondant à ladite première unité de communication (14), chaque deuxième unité de communication (44) étant, le cas échéant, propre à communiquer avec la première unité de communication (14) de l'élément (12) reçu dans l'emplacement (58) en vis-à-vis de ladite deuxième unité de communication (44) pour obtenir des informations relatives à l'élément (12), le dispositif comprenant, en outre, une unité de traitement (33) connectée à chaque deuxième unité de communication (44), l'unité de traitement (33) étant propre à déterminer, à partir desdites informations communiquées, l'occupation de chaque emplacement (58) et, le cas échéant, un état de l'élément (12) reçu dans ledit emplacement (58), l'unité de traitement (33) étant propre à déclencher une alarme en fonction de l'occupation de chaque emplacement (58) et, le cas échéant, de l'état de l'élément (12) correspondant audit l'emplacement (58).

9. Installation (10) comprenant :
- une enceinte (20) comprenant un compartiment interne (24), et
- un dispositif (22) selon l'une quelconque des revendications 1 à 8, le dispositif (22) étant disposé dans le compartiment interne (24) de l'enceinte (20).

10. Installation (10) selon la revendication 9, dans laquelle l'enceinte (20) est une enceinte réfrigérante.

11. Installation (10) selon la revendication 9, dans laquelle les éléments (12) sont des conteneurs de plaquettes, l'enceinte (20) étant un agitateur de plaquettes.

## Patentansprüche

1. Vorrichtung (22) zum Lagern von Elementen (12), wobei jedes Element (12) eine erste drahtlose Kommunikationseinheit (14) aufweist, wobei die Vorrichtung (22) mindestens einen Schubladeblock (30) aufweist, wobei jeder Schubladeblock (30) folgendes aufweist:
- eine Halterung (40) aufweisend eine Aufnahme (45),
- eine Schublade (42), die in der Aufnahme (45) der Halterung (40) angebracht ist und geeignet ist, relativ zur Halterung (40) zu gleiten, wobei die Schublade (42) einen Boden (56) aufweist, der mindestens eine Stelle (58) zur Aufnahme eines Elements (12) definiert,
- für jede Stelle (58) mindestens eine zweite drahtlose Kommunikationseinheit (44), die Funkfrequenzwellen senden kann, wobei die zweite Kommunikationseinheit (44) zur Kommunikation mit der Gesamtheit der ersten Kommunikationseinheiten (14) geeignet ist,
**dadurch gekennzeichnet, dass** der Boden (56) der Schublade (42) aus einem Material gebildet ist, das geeignet ist, von Funkwellen durchquert zu werden, die von der oder jeder zweiten Kommunikationseinheit (44) gesendet werden, wobei die oder jede zweite Kommunikationseinheit (44) unterhalb des Bodens (56) der Schublade (42) gegenüber der korrespondierenden Stelle (58) des Bodens (56) der Schublade (42) angeordnet ist, um die Kommunikation zwischen der zweiten Kommunikationseinheit (44) und der ersten Kommunikationseinheit (14) eines an der Stelle (58) aufgenommenen Elements (12) zu ermöglichen.

2. Vorrichtung (22) gemäß Anspruch 1, wobei der Boden (56) der Schublade (42) aus Kunststoff besteht.

3. Vorrichtung (22) gemäß Anspruch 1 oder 2, wobei die Elemente (12) Behälter für biologische Produkte, Arzneimittel oder therapeutische Zubereitungen sind.

4. Vorrichtung (22) gemäß einem der Ansprüche 1 bis 3, wobei jede erste Kommunikationseinheit (14) ein RFID-Tag ist und jede zweite Kommunikationseinheit (44) ein RFID-Lesegerät ist.

5. Vorrichtung (22) gemäß einem der Ansprüche 1 bis 4, wobei jede zweite Kommunikationseinheit (44) mit der Halterung (40) des korrespondierenden Schubladenblocks (30) fest verbunden ist.

6. Vorrichtung (22) gemäß einem der Ansprüche 1 bis 4, wobei jede zweite Kommunikationseinheit (44) mit der Schublade (42) des korrespondierenden Schubladenblocks (30) fest verbunden ist.

7. Vorrichtung (22) gemäß einem der Ansprüche 1 bis 6, wobei die Vorrichtung (22) eine Mehrzahl an Schubladenblöcken (30) aufweist, wobei ein oberes Ende (46) und/oder ein unteres Ende (48) der Halterung (40) jedes Schubladenblocks (30) ein Verbindungselement (51,52) aufweist mit einem Verbindungselement (51, 52), das jeweils zu einem unteren Ende (48) oder einem oberen Ende (48) der Halterung (40) eines der anderen Schubladenblöcke (30) komplementär ist, sodass die Schubladenblöcke (30) übereinander angeordnet sind, um einen vertikalen Stapel (38) zu bilden.

8. Vorrichtung (22) gemäß einem der Ansprüche 1 bis 7, wobei jede erste Kommunikationseinheit (14) Informationen bezüglich des zu der jeweiligen ersten Kommunikationseinheit (14) korrespondierenden Elements (12) aufweist, wobei jede zweite Kommunikationseinheit (44) geeignet ist, um mit der ersten Kommunikationseinheit (14) des an der Stelle (58) gegenüber der zweiten Kommunikationseinheit (44) aufgenommenen Elements (12) zu kommunizieren, um Informationen bezüglich des Elements (12) zu erhalten, wobei die Vorrichtung ferner eine Verarbeitungseinheit (33) aufweist, die mit jeder zweiten Kommunikationseinheit (44) verbunden ist, wobei die Verarbeitungseinheit (33) geeignet ist, um aus den übermittelten Informationen die Belegung jeder Stelle (58) und gegebenenfalls einen Zustand des an der Stelle (58) aufgenommenen Elements (12) zu bestimmen, wobei die Verarbeitungseinheit (33) geeignet ist, um einen Alarm in Abhängigkeit von der Belegung jeder Stelle (58) und gegebenenfalls dem Zustand des zu der jeweiligen Stelle (58) korrespondierenden Elements (12) auszulösen.

9. Anlage (10) aufweisend:
- ein Gehäuse (20), das ein Innenfach (24) aufweist, und
- eine Vorrichtung (22) gemäß einem der Ansprüche 1 bis 8, wobei die Vorrichtung (22) in dem Innenfach (24) des Gehäuses (20) angeordnet ist.

10. Anlage (10) gemäß Anspruch 9, wobei das Gehäuse (20) ein Kühlgehäuse ist.

11. Anlage (10) gemäß Anspruch 9, wobei die Elemente (12) Blutplättchenbehälter sind, wobei das Gehäuse (20) ein Blutplättchenrührer ist.

## Claims

1. Device (22) for storing elements (12), each element (12) comprising a first wireless communication unit (14), the device (22) comprising at least one drawer assembly (30), each assembly drawer (30) comprising:
- a support (40) comprising a housing (45),
- a drawer (42) positioned in the housing (45) of the support (40) and slidable relative to the support (40), the drawer (42) comprising a bottom (56) defining at least one slot (58) for receiving an element (12),
- for each slot (58), at least one second wireless communication unit (44) capable of transmitting radio frequency waves, the second communication unit (44) being designed to communicate with all the first communication units (14),
**characterized in that** the bottom (56) of the drawer (42) is made of a material able to be traversed by radiofrequency waves emitted by the or each second communication unit (44), the or each second communication unit (44) being arranged under the bottom (56) of the drawer (42) facing the corresponding slot (58) of the bottom (56) of the drawer (42) in order to allow communication between the second communication unit (44) and the first communication unit (14) of an element (12) received in the slot (58).

2. Device (22) according to claim 1, wherein the bottom (56) of the drawer (42) is made of plastic.

3. Device (22) according to claim 1 or 2, wherein the elements (12) are containers of biological products, drugs or therapeutic preparations.

4. Device (22) according to any one of the claims 1 to 3, wherein each first communication unit (14) is a radio identification tag and each second communication unit (44) is a radio identification reader.

5. Device (22) according to any one of the claims 1 to 4, wherein each second communication unit (44) is secured to the support (40) of the corresponding drawer assembly (30).

6. Device (22) according to any one of the claims 1 to 4, wherein each second communication unit (44) is secured to the drawer (42) of the corresponding drawer assembly (30).

7. Device (22) according to any one of the claims 1 to 6, wherein the device (22) comprises a plurality of drawer assemblies (30) with an upper end (46) and/or a lower end (48), wherein the support (40) of each slide assembly (30) comprises an assembly member (51, 52) with a respective complementary assembly member (51, 52) of a lower end (48) or an upper end (48) of the support (40) of another of the drawer assemblies (30), so that the drawer assemblies (30) may be assembled on each other to form a vertical stack (38).

8. Device (22) according to any one of the claims 1 to 7, wherein each first communication unit (14) comprises information relative to the element (12) corresponding to said first communication unit (14), wherein each second communication unit (44) is, if appropriate, able to communicate with the first communication unit (14) of the element (12) received in the slot (58) opposite the second communication unit (44) for obtaining information relative to the element (12), wherein the device further comprises a processing unit (33) connected to each second communication unit (44), wherein the processing unit (33) is able to determine, from the communicated information, the occupation of each slot (58) and, if appropriate, the state of the element (12) received in the slot (58), wherein the processing unit (33) is able to trigger an alarm according to the occupation of each slot (58) and, if appropriate, the state of the element (12) corresponding to the slot (58).

9. Installation (10) comprising:
- an enclosure (20) comprising an internal compartment (24), and
- a device (22) according to any one of the claims 1 to 8, wherein the device (22) is arranged in the inner compartment (24) of the enclosure (20).

10. Installation (10) according to claim 9, wherein the enclosure (20) is a refrigerating enclosure.

11. Installation (10) according to claim 9, wherein the elements (12) are platelet containers, while the enclosure (20) is a platelet stirrer.
